# EUROPEAN PATENT SPECIFICATION

(11) **EP 4 514 307 B1**
(45) Date of publication and mention of the grant of the patent: **03.09.2025**
(21) Application number: 23720075.3
(22) Date of filing: 14.04.2023
(51) Int. Cl.: A61K 8/06, A61K 8/34, A61K 8/37, A61K 8/73, A61Q 1/00, A61Q 19/00, A61K 8/42

(54) **AERATED COSMETIC COMPOSITION**
BELÜFTETE KOSMETISCHE ZUSAMMENSETZUNG
COMPOSITION COSMÉTIQUE AÉRÉE

(30) Priority: 29.04.2022 US 202263336374 P; 16.05.2022 EP 22173451
(43) Date of publication of application: 05.03.2025
(73) Proprietor: Unilever IP Holdings B.V., 3013 AL Rotterdam (NL); Unilever Global IP Limited, Wirral, Merseyside CH62 4ZD (GB)
(72) Inventor: ALTAMURA, Michael Brandon, 6708 WH Wageningen (NL); PEREIRA, Krista Roman, 6708 WH Wageningen (NL); SMITH, Evan Lynn, 6708 WH Wageningen (NL)
(74) Representative: James, Helen Sarah
(86) International application number: PCT/EP2023/059852
(87) International publication number: WO 2023/208618

(56) References cited:
- WO-A1-2015/096115
- WO-A1-2015/097594
- FR-A1- 3 094 221

## Description

### Field of the Invention

Disclosed herein is a personal care composition. More particularly, disclosed herein is a cosmetic composition comprising a starch, a polyol, and a nonionic emulsifier, wherein the composition is suitable for topical application to skin, is an oil-in-water emulsion aerated with air and/or an inert gas.

### Background of the Invention

Many consumers gravitate towards cosmetic compositions said to have light or whipped textures, which are formulated to provide for a pleasant use and post-use experience upon application. Such compositions also tend to more easily be spread upon and/or penetrate the skin. Post-application, such compositions do not leave a greasy or heavy sensory on the skin, while still conferring a moisturization benefit on the skin.

Cosmetic compositions with light or whipped textures are often aerated, meaning that a gas of choice, such as air, has been incorporated into the base composition. However, an aerated cosmetic composition tends to be relatively unstable due to its low density and propensity for separation. These are very challenging problems to be addressed for aerated cosmetic compositions.

Therefore, the present inventors have recognized a need to develop a stable, aerated cosmetic composition in the form of a low-density emulsion. Thus, disclosed herein is a cosmetic composition having starch, a polyol, nonionic emulsifier, an aqueous phase, and an oil phase, wherein the composition is suitable for topical application to skin, is an oil-in-water emulsion, and is aerated with air and/or an inert gas. Such compositions are sustainable because, surprisingly, excellent sensory characteristics and skin benefits are provided to the consumer after use whereby less product than that typically used per single application with conventional cosmetic compositions.

### Additional Information

Efforts have been disclosed for cosmetic compositions incorporating air or gas.

U.S. Patent No. 8,114,855 discloses a cosmetic mixture incorporating a gas, wherein the cosmetic mixture comprises an alk(en)ylpolyglycoside, a quaternary ammonium polymer, and a thickener, for forming low density formulations.

U.S. Patent No. 9,193,852 relates to a composition comprising an oily phase dispersed in an aqueous phase, characterized in that the composition contains air or inert gas, at least one modified starch, at least one emulsifier, and at least one oil-dispersible structuring agent.

PCT Publication No. 2015/096115 describes an oil-in-water composition in the form of a foam having a dispersed fatty phase in a continuous aqueous phase having a density of 0.3 to 0.95 comprising at least one starch phosphate, a superabsorbent polymer, a hydrophilic gelling polymer, and a surfactant.

PCT Publication No. 2019/129683 discloses a whipped cosmetic composition in the form of an oil-in-water nanoemulsion comprising liquid fatty substance, cationic surfactant, gas, and water for use in cosmetic treatment of keratin materials.

None of the additional information above describes the particular combination as disclosed and claimed herein.

### Summary of the Invention

The present inventors have found a stable, aerated cosmetic composition in the form of an emulsion having a low density.

The composition relates to a cosmetic composition in the form of an oil-in-water emulsion having a low density comprising starch, a polyol, nonionic emulsifier, an aqueous phase, and an oil phase, wherein the composition is aerated with air and/or an inert gas as defined in claims is obtained by a method of aerating a cosmetic composition comprising the steps of:
(a) Preparing an oil-in-water emulsion comprising starch, polyol, nonionic emulsifier, an aqueous phase, and an oil phase; and
(b) Incorporating air into the cosmetic composition by whipping and/or injection techniques at a temperature ranging from 22°C to 85°C and at a pressure from 0.066 to 2.10 atm (50 to 1600 mmHg), preferably, 0.079 to 1.71 atm (60 to 1300 mmHg), more preferably, 0.092 to 1.315 atm (70 to 1000 mmHg), into a pipe having a diameter ranging from 1.27 to 10.16 cm (0.5 to 4 inches).

There is provided a use of a cosmetic composition of the first aspect for delivering a benefit to the skin.

There is provided a method of manufacturing the cosmetic composition of the first aspect.

The present inventors have unexpectedly found that the compositions disclosed herein are sustainable because excellent sensory and skin benefits are provided to the consumer after use and with less product than typically needed with conventional cosmetic compositions used for similar applications. In addition, the disclosed cosmetic compositions possess a lower density when compared to conventional leave-on compositions but are surprisingly stable and robust enough to support high gas incorporation.

All other aspects of the present cosmetic compositions will become more readily apparent upon considering the detailed description and examples which follow.

### Detailed Description of the Invention

The disclosed cosmetic composition relates to a cosmetic composition that is aerated as defined in claims, wherein
the composition has dispersed therein gas bubbles, and wherein the gas bubbles form from 5 to 35%, or 7 to 30%, or 10 to 25% of the volume of the composition.

The gas bubbles are from a gas such as air, carbon dioxide, nitrogen, nitrous oxide, or mixtures thereof. In another aspect, the gas comprises air, nitrogen, or a mixture thereof.

Cosmetic compositions disclosed herein possess a low density ranging from 0.5 to 1.3 g/cm³, or 0.6 to 1.1 g/cm³, or 0.7 to 1.0 g/cm³, or 0.8 to 0.95 g/cm³, or 0.8 to 0.9 g/cm³ when measured at 25°C and atmospheric pressure. Density of the aerated samples may be measured with a pycnometer, density meter, or via other standard methods known to a skilled artisan.

The polyol compounds comprised in the disclosed cosmetic composition are polyhydric alcohol-type materials. Illustrative but nonlimiting polyhydric alcohols include glycerol (i.e., glycerine or glycerin), propylene glycol, dipropylene glycol, polypropylene glycol, polyethylene glycol, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. In an aspect of the disclosed cosmetic composition, the polyol comprises glycerin, butylene glycol, sorbitol, or a mixture thereof. The amount of polyol employed may range from 0.1 to 15%, or 1 to 12%, or 3 to 10% by weight of the cosmetic composition.

Cosmetic compositions as disclosed herein comprise a starch. Starches as used herein include native and modified (e.g., physically or chemically modified) starches. In one aspect, the starch is a chemically modified starch. Illustrative but nonlimiting examples of native starches include *Maranta arundinacea* (Arrowroot) powder, *Manihot esculenta* (Cassava) starch, *Manihot esculenta* (Tapioca) starch, *Zea mays* (Corn) starch, *Oryza sativa* (Rice) starch, *Solanum tuberosum* (Potato) starch, *Triticum vulgare* (Wheat) starch, *Cycas revoluta* (Sago) starch, *Hordeum vulgare* (Barley) starch, or mixtures thereof. With respect to modified starches, physical modifications may include, in an illustrative but nonlimiting manner, annealing, freeze-thaw treatment, heat treatment (e.g., extrusion heating, simple oven heating, superheating), heat moisture treatment, hydrothermal treatment, mechanical treatment (e.g., grinding, mechanical activation, micronization, milling), moisture treatment, pH treatment, pressure treatment (e.g., dynamic pulsed pressure treatment, high pressure treatment, instantaneous controlled pressure treatment, osmotic pressure treatment, hydrostatic pressure treatment), pulsed-electric field treatment, radiation treatment (e.g., electromagnetic irradiation, gamma irradiation, microwave irradiation, UV irradiation), sonication and ultrasonic treatment, or a combination of different physical factors (e.g., duel modification). Desirable chemical modifications to starches include, for example, techniques known by one of ordinary skill in the art to result in cationization, crosslinking, esterification (e.g., acetylation, fatty acetylation, phosphorylation, succinylation), etherification (e.g., methylation, hydroxymethylation, hydroxypropylation, carboxymethylation), graft copolymerization, hydrolysis, oxidation, or dual modification. Representative of the starches are chemically modified starches such as sodium hydroxypropyl starch phosphate, distarch phosphate, sodium polyacrylate starch, glyceryl starch, and aluminum starch octenylsuccinate. Genetic variants of desirable plant-based starch, such as high amylose and high amylopectin starch, are also in scope for use in the disclosed cosmetic composition. As to starch used in the cosmetic composition, 0.1 to 3%, or 0.25 to 2%, or 0.5 to 1.75%, or 0.75 to 1.6% by weight starch may be used in the cosmetic composition. In an aspect of the disclosed cosmetic composition, the starch comprises sodium hydroxypropyl starch phosphate, tapioca starch, or a mixture thereof.

Cosmetic compositions as disclosed herein are emulsions. In an aspect of the disclosed composition, the emulsion is an oil-in-water emulsion comprising an aqueous phase and an oil phase. The aqueous phase is in an amount of between about 30 to 80%, or 35 to 75%, or 40 to 70% by weight of the aerated cosmetic composition, including all ranges subsumed therein. In one aspect, the aqueous phase comprises water. In another aspect, the aqueous phase consists essentially of water.

Desirable emulsions have an oil phase comprising at least one oil, butter, wax, or a mixture thereof. Illustrative but nonlimiting examples of oils include *Adansonia digitata* (Baobab) seed oil, *Argania spinosa* (Argan) kernel oil, behenyl neopentanoate, *Borago officinalis* (Borage) seed oil, *Brassica campestris* (Rapeseed) seed oil, *Calophyllum inophyllum* (Tamanu) seed oil, *Camelina sativa* (False Flax) seed oil, *Camellia oleifera* (Camellia) seed oil, caprylic/capric triglyceride, *Carthamus tinctorius* (Safflower) seed oil, coco-caprylate/caprate, *Cocos nucifera* (Coconut) oil, *Corylus avellana* (Hazel) seed oil, *Crambe abyssinica* (Crambe) seed oil, *Gossypium herbaceum* (Cotton) seed oil, *Helianthus annuus* (Sunflower) seed oil, *Hippophae rhamnoides* (Sea Buckthorn) oil, isopropyl myristate, isononyl isonanonoate, isopropyl palmitate, jojoba esters, lanolin oil and lanolin derivatives, *Limnanthes Alba* (Meadowfoam) seed oil, *Linum usitatissimum* (Linseed) seed oil, *Macadamia ternifolia* (Macadamia) seed oil, *Moringa oleifera* (Moringa) seed oil, *Oenothera biennis* (Evening Primrose) oil, *Olea europaea* (Olive) fruit oil, *Orbignya oleifera* (Babassu) seed oil, *Oryza sativa* (Rice) germ oil, *Persea gratissima* (Avocado) oil, *Plukenetia volubilis* (Sacha inchi) seed oil, *Prunus amygdalus dulcis* (Sweet Almond) oil, *Ricinus communis* (Castor) seed oil, *Rosa canina* (Rosehip) seed oil, *Salvia hispanica* (Chia) seed oil, *Salvia officinalis* (Sage) oil, *Schinziophyton rautanenii* (Mongongo) kernel oil, *Sclerocarya birrea* (Marula) seed oil, *Sesamum indicum* (Sesame) seed oil, *Shorea Robusta* (Sal) seed oil, silicone, *Simmondsia chinensis* (Jojoba) seed oil, squalane, *Thymus vulgaris* (Thyme) oil, Triolein (algae oil), *Triticum vulgare* (Wheat) germ oil, *Vitis vinifera* (Grapeseed) oil, *Zea mays* (Corn) oil, and mixtures thereof.

The cosmetic compositions disclosed herein may comprise a butter, including a plant-based butter. The term butter is understood by one of ordinary skill in the art and means a triglyceride which has the consistency of butter. Illustrative but nonlimiting butters desirable for use include aloe butter, avocado butter, bacuri butter, cocoa butter, coconut butter, coffee bean butter, cupuaçu butter, hemp seed butter, illipe butter, kokum butter, macadamia nut butter, mango butter, mochacchino butter, murumuru butter, olive butter, pistachio nut butter, refined butter, shea butter, sweet almond butter, tucuma butter, and ucuuba butter.

Waxes and wax esters are often desirable for use in the disclosed cosmetic composition. These waxes include petroleum-derived waxes (e.g., paraffin wax, petrolatum, microcrystalline wax), animal-derived waxes or those from animal byproducts (e.g., beeswax, spermaceti wax, Chinese wax, wool wax, shellac wax), plant-derived waxes (e.g., tribehenin wax, carnauba wax, candelilla wax, bayberry wax, jojoba wax, orange wax, rice bran wax, rose flower wax, sunflower wax, castor wax, soy wax), mineral waxes (e.g., montan wax, ceresin wax, ozokerite) and synthetic derivatives of natural waxes.

The oil, butter, wax, or mixture thereof may be incorporated in the inventive composition in total amounts ranging from 20 to 50%, or 25 to 45%, or 30 to 40%, or 31 to 37% by weight of the composition. In another aspect, it is provided that the oil, butter, wax or mixture thereof is selected from comprises candelilla wax, caprylic/capric triglyceride, carnauba wax, cocoa butter, coconut oil, jojoba seed oil, shea butter, and sunflower oil. In one aspect of the disclosed cosmetic composition, at least 30%, or at least 40% of the oil, butter, or wax is solid at room temperature.

In an aspect of the disclosed cosmetic composition, the composition is natural, or naturally derived.

The cosmetic composition disclosed herein comprises an emulsifier conventionally acceptable for cosmetic use in oil-in-water emulsions. The emulsifier disclosed comprises at least a nonionic emulsifier. Illustrative but nonlimiting nonionic emulsifiers are those with a C₁₀-C₂₀ fatty alcohol or acid hydrophobe condensed with 2 to 100 moles of ethylene oxide or propylene oxide per mole of hydrophobe (e.g., cetyl alcohol, stearyl alcohol); C₂-C₁₀ alkyl phenols condensed with 2 to 20 moles of alkylene oxide; mono- and di-fatty acid esters of ethylene glycol; fatty acid monoglyceride; sorbitan, mono- and di- C₈-C₂₀ fatty acids; and polyoxyethylene sorbitan, as well as combinations thereof. Other illustrative but nonlimiting nonionic emulsifiers include glyceryl stearate and glycol stearate with stearamide AMP. In one aspect, the disclosed cosmetic compositions are substantially free of alk(en)ylpolyglycosides and derivatives thereof. Substantially free of alk(en)ylpolyglycosides means that the composition contains less than 0.5%, or less than 0.1%, or less than 0.01% by weight, or 0% by weight alk(en)ylpolyglycosides.

The disclosed cosmetic composition is a leave-on composition intended to be applied to remain on the skin. The disclosed cosmetic composition predominantly contains, with respect to emulsifiers, nonionic emulsifiers. Optionally, the cosmetic composition may further comprise anionic, cationic, amphoteric or zwitterionic emulsifiers. Preferred anionic emulsifiers include alkyl ether sulfate and sulfonates, alkyl sulfates and sulfonates, alkylbenzene sulfonates, alkyl and dialkyl sulfosuccinates, C₈-C₂₀ acyl isethionates, C₈-C₂₀ alkyl ether phosphates, alkylethercarboxylates and combinations thereof.

Cationic emulsifiers that may be used include, for example, palmitamidopropyltrimonium chloride, distearyldimonium chloride and mixtures thereof. Useful amphoteric emulsifiers include cocoamidopropyl betaine, C₁₂-C₂₀ trialkyl betaines, sodium lauroamphoacetate, and sodium laurodiamphoacetate or a mixture thereof.

In one aspect, the cosmetic composition comprises 0.1 to 10% emulsifier by weight of the cosmetic composition. In another aspect, the cosmetic composition comprises 0.5 to 8%, or 1 to 7% emulsifier by weight of the composition. In still another aspect, the cosmetic composition comprises 3 to 6% emulsifier by weight of the composition. In yet another aspect, the cosmetic composition comprises 5 to 7% emulsifier by weight of the composition. It is within the scope of the disclosed cosmetic compositions to use combinations of emulsifiers wherein the combined HLB value overall ranges from 5 to 15, or 6 to 13. The overall HLB of the emulsifier or emulsifiers used when water-continuous emulsions are desired will be 7 to 11, or from 8 to 10. In one aspect, the nonionic emulsifier comprises glyceryl stearate, glycol stearate and stearamide AMP, PEG-100 stearate, cetyl alcohol, stearyl alcohol, or mixtures thereof.

The weight ratio of total amount of emulsifier to the oil phase is 1: 6.5 to 1: 3.75, or 1: 6.25 to 1: 3.8, or 1:6 to 1:4.

Now, it has unexpectedly been determined that the cosmetic compositions disclosed herein may be substantially free of thickening agent. Substantially free of thickening agent means that the composition contains less than 0.2%, or less than 0.1%, or less than 0.05%, or less than 0.01%, or 0% by weight thickening agent. Conventional thickening agents include water-dispersible polysaccharides such as acacia senegal gum, agar, agarose, biosaccharide gum, carrageenan, cellulose, gellan gum, guar gum, hyaluronic acid, pectin, sclerotium gum, and xanthan gum; polymeric thickeners such as polyacrylates, polyacrylamides, polymethacrylate, acrylate copolymer, methacrylate copolymer, acrylamide copolymer, and acryloyldimethyltaurate copolymer; and mixtures thereof.

One of ordinary skill in the art would be adept to identify any need of neutralization. Any pH modifier known in the field acceptable for use in cosmetic and/or pharmaceutical products can be employed to result in a cosmetic composition possessing a skin-acceptable pH, typically within the range of 4 to 8, where pH is determined using a Thermo Fisher Scientific pH meter. In an aspect of the disclosed composition, the pH of the cosmetic composition is 4.25 to 7.5, or 4.5 to 7, or 5 to 6. Traditional buffers or pH modifiers include common additives such as sodium hydroxide, potassium hydroxide, hydrochloric acid, citric acid, triethanolamine, and aminomethyl propanol.

Viscosity of the cosmetic composition disclosed herein is 1,000 to 50,000 centipoise (cps), or 5,000 to 40,000 cps, taken under conditions of ambient temperature and a shear rate of 10s⁻¹ with a strain controlled parallel plate rheometer made commercially available from suppliers like T.A. Instruments under the Discovery name. Alternatively, viscosity can also be measured using a Brookfield Viscometer (speed at 20 rpm, spindle 5, helipath off, for one (1) minute at ambient temperature). In an aspect of the disclosed cosmetic composition, the composition has a viscosity of 9,000 to 25,000 cps, or 10,000 to 22,000 cps (1cps= 1 mPa.s)

Optional ingredients that may be used in the disclosed cosmetic composition include preservatives to assist against the growth of potentially harmful microorganisms when the cosmetic composition is made. Traditional preservatives that may be used include hydantoin derivatives, propionate salts, and a variety of quaternary ammonium compounds. Other preservatives include potassium sorbate, iodopropynyl butyl carbamate, phenoxyethanol, methyl paraben, wasabi-based preservatives, propyl paraben, imidazolidinyl urea, sodium gluconate, sodium benzoate, sodium dehydroacetate, and benzyl alcohol. Especially preferred additives desirable to be employed in the disclosed cosmetic composition are 1,2-alkanediols like 1,2-octanediol, 1,2 hexanediol, or mixtures thereof. In one aspect, the cosmetic composition may comprise of a preservative system that is formaldehyde-free, paraben-free, or both. In another aspect, the cosmetic composition may be free of preservative. In yet another aspect, the cosmetic composition is substantially free of silicones, sulfates, or a combination thereof. In still another aspect, the cosmetic composition does not contain silicone, sulfate, or both.

Traditional fragrance components like eugenol, coumarin, linalyl acetate, citronellal, iris concentrate, terpinyl acetate, terpineol, thymol, pinenes (e.g., alpha and beta pinene) and citronellol may optionally be added to the cosmetic composition as well.

If employed, the traditional preservatives, vicinal diol and/or fragrance component will not make up more than 2%, or not more than 1% by weight of the cosmetic composition. In one aspect, from 0.2 to 0.85%, or 0.2 to 0.8% by weight optional preservative, vicinal diol and/or fragrance component is used, based on total weight of the cosmetic composition.

Additional optional ingredients that may be used include sensory oils and/or exfoliants. Desirable oils include rose, lime, coconut, lavender, argan, sweet almond oil(s) or mixtures thereof. Illustrative exfoliants desirable for use include salt, sugar, apricot, walnut shell, rice, nutmeg and/or oatmeal powder(s). When used, sensory oils and exfoliants can make up from 0.1 to 2% by weight of the cosmetic composition, with the proviso that the total amount of fragrance and sensory oil does not exceed 2.5% by weight of the cosmetic composition, or not more than 2% by weight of the composition.

The cosmetic composition disclosed herein may include vitamins. Illustrative vitamins are Vitamin B₂, Vitamin B₃ (niacinamide), Vitamin B₆, Vitamin C, Vitamin E, Folic Acid and Biotin. Derivatives of the vitamins may also be employed. For instance, Vitamin C derivatives include ascorbyl tetraisopalmitate, magnesium ascorbyl phosphate and ascorbyl glycoside. Derivatives of Vitamin E include tocopheryl acetate, tocopheryl palmitate and tocopheryl linoleate. DL-panthenol and derivatives may also be employed. Total amount of vitamins when present may range from 0.001 to 10%, or 0.01% to 5%, or 0.1 to 3% by weight of the cosmetic composition.

Other optional additives desirable for use include resorcinols like 4-ethyl resorcinol, 4-hexyl resorcinol, 4-phenylethyl resorcinol, dimethoxytoluyl propyl resorcinol, 4-cyclopentyl resorcinol, 4-cyclohexylresorcinol, thiamidol; alpha- and/or beta-hydroxyacids; retinoic acid and its derivatives (e.g., cis and trans); retinal; retinol; retinyl esters such as retinyl acetate, retinyl palmitate, and retinyl propionate; petroselinic acid; conjugated linoleic acid; mixtures thereof or the like. Still other optional additives like ethanol, quaternary ammonium compounds (like cetrimonium chloride, benzalkonium chloride or the like) and may also be included. Such additives, when used, collectively make up from 0.001 to 3%, or 0.01 to 2%, or 0.1 to 1.5% by weight of the cosmetic composition.

Desquamation promoters may be present. Illustrative are the alpha-hydroxycarboxylic acids, beta-hydroxycarboxylic acids. The term "acid" is meant to include not only the free acid but also salts and C₁-C₃₀ alkyl or aryl esters thereof and lactones generated from removal of water to form cyclic or linear lactone structures. Representative acids are glycolic and its derivatives, lactic and malic acids. Amounts of these materials when present may range from 0.01 to 3%, or from 0.1 to 2% by weight of the cosmetic composition.

Another optional additive suitable for use includes hemp oil with 2.5 to 25% by weight cannabigerol and/or cannabidiol at from 0.5 to 10 percent by weight. When used, such oil makes up from 0.0001 to 12%, or from 0.01 to 5% by weight of the cosmetic composition.

Also optionally the disclosed cosmetic composition may include materials like chelators (e.g., EDTA), opacifiers (like TiO₂, particle size from 50 to 1200 nm, or 50 to 350 nm), kaolin, bentonite, zinc oxide, iron oxide, mica, C₈₋₂₂ fatty acid substituted saccharides, lipoic acid, retinoxytrimethylsilane (available from Clariant Corp. under the Silcare 1M-75 trademark), dehydroepiandrosterone (DHEA), or mixtures thereof. Ceramides (including Ceramide 1, Ceramide 3, Ceramide 3B and Ceramide 6) as well as pseudoceramides may also be optionally included as can 10- and/or 12-hydroxystearic acid. Amounts of these optional materials when used may range from 0.0001 to 3%, or from 0.001 to 2%, or from 0.001 to 1.5% by weight of the cosmetic composition.

Colorants or dyes may also be included in the disclosed compositions. These substances may range from 0.05 to 5%, or between 0.1 to 2% by weight of the composition.

Sunscreen actives may also be optionally included in the disclosed cosmetic composition. Particularly desired are such materials as ethylhexyl p-methoxycinnamate, available as Parsol MCX^{®}, Avobenzene, available as Parsol 1789^{®} and benzophenone-3, also known as Oxybenzone. Inorganic sunscreen actives may be employed such as microfine titanium dioxide, zinc oxide, polyethylene, and various other polymers. Amounts of the sunscreen agents when present may generally range from 0.01 to 3%, or from 0.5 to 2%, or 0.75 to 1.5% by weight of the cosmetic composition.

A cosmetic composition as disclosed herein comprises:
a. a starch;
b. a polyol;
c. a nonionic emulsifier;
d. an aqueous phase; and
e. an oil phase,

wherein the cosmetic composition is aerated with air and/or an inert gas,
and further wherein the cosmetic composition is an oil-in-water emulsion that is topically applied to skin.

In one aspect, a cosmetic composition as disclosed herein comprises:
a. a starch;
b. a polyol;
c. a nonionic emulsifier;
d. an aqueous phase; and
e. an oil phase,

wherein the cosmetic composition is aerated with air and/or an inert gas,
wherein the cosmetic composition is substantially free of alk(en)ylpolyglycosides, thickening agent, or mixtures thereof,
and further wherein the cosmetic composition is an oil-in-water emulsion that is topically applied to skin.

In another aspect, a cosmetic composition as disclosed herein comprises:
a. 0.5 to 5% by weight of a starch;
b. 0.1 to 15%, or 1 to 12% by weight of polyol;
c. 5 to 7% by weight of a nonionic emulsifier;
d. an aqueous phase; and
e. an oil phase,

wherein the cosmetic composition is aerated with air, nitrogen, or a mixture thereof, the cosmetic composition has a density ranging from 0.7 to 1.0 g/cm³,
further wherein the cosmetic composition is an oil-in-water emulsion that is topically applied to skin and is substantially free of alk(en)ylpolyglycosides, thickening agent, or mixtures thereof, and the weight ratio of total amount of emulsifier to oil phase is 1: 6.5 to 1: 3.75, preferably, 1: 6.25 to 1: 3.8, more preferably, 1:6 to 1:4.

In yet another aspect, a cosmetic composition as disclosed herein comprises:
a. 0.5 to 5% by weight of a chemically modified starch;
b. 0.1 to 15%, or 1 to 12% by weight of polyol;
c. 5 to 7% by weight of a nonionic emulsifier;
d. an aqueous phase; and
e. an oil phase,

wherein the cosmetic composition is aerated with air, nitrogen, or a mixture thereof, the cosmetic composition has a density ranging from 0.7 to 1.0 g/cm³,
wherein the cosmetic composition is an oil-in-water emulsion that is topically applied to skin and is substantially free of alk(en)ylpolyglycosides, thickening agent, or mixtures thereof,
and further wherein the weight ratio of total amount of emulsifier to the emulsion's oil phase is 1: 6.5 to 1: 3.75, preferably, 1: 6.25 to 1: 3.8, more preferably, 1:6 to 1:4.

In still another aspect, a cosmetic composition as disclosed herein comprises:
a. 0.5 to 5% by weight of a hydroxypropyl starch phosphate, tapioca starch, or a mixture thereof;
b. 0.1 to 15%, preferably, 1 to 12% by weight of polyol; and
c. 5 to 7% by weight of a nonionic emulsifier with an average HLB value of 7 to 11;
d. an aqueous phase; and
e. an oil phase,

wherein the cosmetic composition is aerated with air, nitrogen, or a mixture thereof,
wherein the cosmetic composition has a density ranging from 0.7 to 1.0 g/cm³,
wherein the cosmetic composition is an oil-in-water emulsion that is topically applied to skin and is substantially free of alk(en)ylpolyglycosides, thickening agent, and mixtures thereof,
and further wherein the weight ratio of total amount of emulsifier to the emulsion's oil phase is 1: 6.5 to 1: 3.75, preferably, 1: 6.25 to 1: 3.8, more preferably, 1:6 to 1:4.

The disclosed cosmetic composition also relates to the process for manufacturing the aerated composition. The process involves preparing an emulsion by combining an oil phase, an aqueous phase, and emulsifier under conditions of moderate sheer at 22°C to 85°C to produce an oil-in-water emulsion, and then introducing gas into the emulsion to aerate the composition at a pressure ranging from 50 to 1600 mmHg, or 60 to 1300 mmHg, or 70 to 1000 mmHg, or 80 to 500 mmHg, 100 to 300 mmHg, and a temperature of 22 to 85°C, or 30 to 70°C, or 35 to 60°C, into a pipe having a diameter ranging from 0.5 to 4 inches. In one aspect, the introduction of gas occurs by whipping/mixing. In another aspect, the introduction of gas occurs by injection (1mmHg= 0.0013 atm).

Many types of packaging can be used to store and deliver the present cosmetic composition. The selection of packaging is dependent upon the personal care end-use and the viscosity of the composition itself. As an example, leave-on lotions and creams for skin typically employ plastic containers with an opening at a dispense end covered by an appropriate closure. Conventional closures include flip-top hinged lids, screw-caps and non-aerosol pumps. As another example, appropriate packaging to be used for antiperspirants, deodorants and depilatories include a container with a roller-ball applicator on a dispensing end if the composition is fluid and of a thinner viscosity. In general, patches, bottles, tubes, roller-ball applicators, squeeze containers or lidded jars are preferred.

For the avoidance of doubt, the term "comprising" is meant not to be limiting to any stated elements but rather to encompass non-specified elements of major or minor functional importance. Therefore, the listed steps, elements or options need not be exhaustive. Whenever the words "including" or "having" are used, these terms are meant to be equivalent to "comprising" as defined above.

The term "skin" as used herein includes the skin on the face, neck, chest, back, arms, axilla, buttocks, hands, legs and scalp. Skin benefit agent, as used herein, is meant to include a component that improves a facial or body characteristic after topical application like a skin characteristic and/or benefits the same wherein the skin benefit agent can be, or is, a skin benefit agent in a cream, serum, lotion, balm, deodorant, or non- or low-foaming wash composition. In one embodiment, the composition is a leave-on composition. The cosmetic composition includes conditioners, lotions, creams, balms, deodorant, shaving creams, masks, and non-foaming cleansers. The term "aerated" means that gas has been intentionally incorporated into the composition, for example, by mechanical means. The terms "aeratable" means that gas can be incorporated into the composition, for example, by whipping or injection techniques. As used herein, "stable" refers to a composition that is not visually observed to have separation after being subjected to conditions of 50°C at 2 weeks, or at 4 weeks. As used herein, "aqueous phase" means a phase having at least 80%, or 90%, or 100% by weight water.

All states of matter, as used herein, such as solid, liquid and gas relate to the specified state of matter at 25°C and atmospheric pressure. Herein, references to densities should be understood to be densities measured at 25°C and atmospheric pressure using standard methods. As used herein, "low density" refers to densities ranging from 0.5 to 1.4 g/cm³, or 0.6 to 1.1 g/cm³, or 0.7 to 1.0 g/cm³, or 0.8 to 0.95 g/cm³, or 0.8 to 0.9 g/cm³ when measured at 25°C and atmospheric pressure. Substantially free of thickening agent, as used herein, means that the composition contains less than 0.2%, or less than 0.1%, or less than 0.05%, or less than 0.01%, or 0% by weight thickening agent. Substantially free of alk(en)ylpolyglycosides, as used herein, means that the composition contains less than 0.5%, or less than 0.1%, or less than 0.01% by weight, or 0% by weight alk(en)ylpolyglycosides.

Except in the examples, or where otherwise explicitly indicated, all numbers in this description indicating amounts of material are to be understood as modified by the word "about." All amounts are by total weight of the composition, unless otherwise specified.

The cosmetic compositions will now be described below in the context of specific non-limiting examples to facilitate a greater understanding of the present cosmetic compositions. One of ordinary skill in the art will recognize that variations of the inventive compositions that differ from the examples given may be practiced without deviating from the teachings of the present compositions.

### Examples

All samples were made by mixing the mentioned ingredients under conditions of moderate sheer, 22°C to 85°C, and atmospheric pressure.

### Example 1: Sample formulations (Examples D-F are comparative examples)

| Ingredient | Weight percent (wt%) | | | | | | |
|---|---|---|---|---|---|---|---|
| | A | B | C | D | E | F | G |
| Water | Balance | Balance | Balance | Balance | Balance | Balance | Balance |
| Glycerin | 8.00 | 8.00 | 8.00 | 8.00 | 8.00 | 8.00 | 8.00 |
| Sodium hydroxypropyl starch phosphate | 1.50 | 1.50 | 1.50 | 1.50 | 1.50 | 1.50 | - |
| Montanov 68 (HLB 9.3) | 3.50 | 3.50 | 3.50 | 3.50 | 3.50 | 3.50 | 3.50 |
| Glyceryl stearate (HLB 3.8) | 1.50 | 1.50 | 1.50 | 1.50 | 1.50 | 1.50 | 1.50 |
| Antioxidant | 0.10 | 0.10 | 0.10 | 0.10 | 0.10 | 0.10 | - |
| Preservative | 0.85 | 1.10 | 1.10 | 1.10 | 1.10 | 1.10 | - |
| pH adjuster | - | 0.08 | 0.08 | 0.08 | 0.08 | 0.08 | - |
| Oil/butter/wax blend (cocoa butter, shea butter, Glycine soja (soybean) oil, isopropyl palmitate, carnauba wax, and coconut oil) | 25.6 | 21.5 | 17.3 | 12.4 | 28.9 | 8.25 | 21.5 |
| Tapioca Starch | 1.55 | 1.30 | 1.05 | 0.75 | 1.75 | 0.50 | 3.00 |
| Sensory oil | 3.88 | 3.25 | 2.63 | 1.88 | 4.38 | 1.25 | - |
| | | | | | | | |
| Visual appearance* | Whipped | Whipped | Whipped | Whipped | Whipped | Whipped | Whipped |
| Viscosity (10s⁻¹ at 25°C, 30 seconds) in mPas (Cps) | 13080 | 11055 | - | - | - | - | 14060 |
| Density (kg/m³) | - | 0.833 | - | - | - | - | 0.902 |
| Emulsifier to total oil phase weight ratio | 1:6 | 1:5 | 1:4 | 1:3 | 1:7 | 1:2.5 | 1:4.9 |
| Stability at 50°C | Stable (at 4wks) | Stable (at 4wks) | Stable (at 4wks) | Unstable (at 4wks) | Unstable (at 4wks) | Unstable (at 2 wks) | - |

Cosmetic compositions were made by combining the above ingredients in accordance with the disclosed cosmetic compositions. Each sample was injected with compressed air to create the aerated product. Samples A to C and sample G illustrate compositions in scope with the disclosed cosmetic composition, which surprisingly resulted in stable compositions with whipped appearances and/or low densities around 0.85 kg/m³. Samples D to F illustrate compositions not made consistent with the disclosed cosmetic compositions, which resulted in unstable compositions that separated and were flat and undesirably compressed.

*Visual appearance and whipped means having an aerated sensory and appearance and feeling lighter and easier to spread than the compositions not made according to the disclosed cosmetic composition as determined by skilled laboratory panelists.

## Claims

1. A cosmetic composition in the form of an aerated oil-in-water emulsion, the cosmetic composition comprising:
a. A starch;
b. A polyol;
c. A nonionic emulsifier;
d. An aqueous phase; and
e. An oil phase,
wherein the weight ratio of total amount of emulsifier to the emulsion's oil phase is 1: 6.5 to 1: 3.75, preferably, 1: 6.25 to 1: 3.8, more preferably, 1:6 to 1:4;
wherein the cosmetic composition has a density when measured at 25°C and atmospheric pressure ranging from 0.5 to 1.3 g/cm³, preferably, 0.6 to 1.1 g/cm³, preferably, 0.7 to 1.0 g/cm³;
wherein the cosmetic composition contains less than 0.2% by weight thickening agent selected from water-dispersible polysaccharides selected from acacia senegal gum, agar, agarose, biosaccharide gum, carrageenan, cellulose, gellan gum, guar gum, hyaluronic acid, pectin, sclerotium gum, and xanthan gum; polymeric thickeners selected from polyacrylates, polyacrylamides, polymethacrylate, acrylate copolymer, methacrylate copolymer, acrylamide copolymer, and acryloyldimethyltaurate copolymer; and mixtures thereof;
wherein the composition has dispersed therein gas bubbles;
wherein the gas bubbles form from 5 to 35%, preferably, 7 to 30%, more preferably, 10 to 25% of the volume of the composition;
wherein the gas bubbles are from air, carbon dioxide, nitrogen, nitrous oxide, or mixtures thereof;
and wherein the composition is obtained by a method comprising the steps of:
a. preparing an oil-in-water emulsion by emulsifying the oil phase into the aqueous phase under conditions of moderate shear at atmospheric pressure and a temperature of 22°C to 85°C; and
b. introducing the gas into the emulsion to aerate the composition by whipping or injection techniques at 0.066 to 2.10 atm (50 to 1600 mmHg) preferably, 0.079 to 1.71 atm (60 to 1300 mmHg), more preferably, 0.092 to 1.315 atm (70 to 1000 mmHg) and a temperature of 22°C to 85°C, into a pipe having a diameter ranging from 1.27 to 10.16 cm (0.5 to 4 inches).

2. The cosmetic composition of Claim 1 , wherein the polyol comprises glycerin, butylene glycol, sorbitol, or a mixture thereof, and wherein the cosmetic composition comprises 0.1 to 15%, preferably, 1 to 12% polyol by weight of the cosmetic composition.

3. The cosmetic composition of any of the preceding claims, wherein the starch is a native starch, chemically modified starch, or mixture thereof.

4. The cosmetic composition of any of the preceding claims, wherein the cosmetic composition comprises 0.1 to 3%, preferably, 0.25 to 2% by weight starch in the cosmetic composition.

5. The cosmetic composition of any of the preceding claims, wherein the cosmetic composition 0.1 to 10%, preferably, 0.5 to 8% nonionic emulsifier by weight of the cosmetic composition and wherein the nonionic emulsifier has an average HLB value of 7 to 11.

6. The cosmetic composition of any of the preceding claims, wherein the nonionic emulsifier comprises glyceryl stearate, glycol stearate and stearamide AMP, PEG-100 stearate, cetyl alcohol, stearyl alcohol, or mixtures thereof.

7. The cosmetic composition of any of the preceding claims, wherein the aqueous phase is in an amount of between 30 to 80%, preferably, 35 to 75%, more preferably, 40 to 70% by weight of the cosmetic composition.

8. The cosmetic composition of any of the preceding claims, wherein the oil phase comprising an oil, butter, wax, or mixture thereof in total amounts ranging from 20 to 50%, preferably, from 25 to 45% by weight of the composition.

9. The cosmetic composition of any of the preceding claims, wherein the oil phase comprises candelilla wax, caprylic/capric triglyceride, carnauba wax, cocoa butter, coconut oil, jojoba seed oil, shea butter, sunflower oil, or mixtures thereof, and wherein at least 30%, preferably, at least 40%, of the oil phase is solid at room temperature.

## Patentansprüche

1. Kosmetische Zusammensetzung in Form einer belüfteten Öl-in-Wasser-Emulsion, wobei die kosmetische Zusammensetzung umfasst:
a. eine Stärke;
b. ein Polyol;
c. einen nichtionischen Emulgator;
d. eine wässrige Phase und
e. eine Ölphase,
wobei das Gewichtsverhältnis der Gesamtmenge des Emulgators zur Ölphase der Emulsion 1:6,5 bis 1:3,75, vorzugsweise 1:6,25 bis 1:3,8, bevorzugter 1:6 bis 1:4 beträgt;
wobei die kosmetische Zusammensetzung eine Dichte, gemessen bei 25°C und Atmosphärendruck, im Bereich von 0,5 bis 1,3 g/cm³, vorzugsweise von 0,6 bis 1,1 g/cm³, vorzugsweise von 0,7 bis 1,0 g/cm³, aufweist;
wobei die kosmetische Zusammensetzung weniger als 0,2 Gew.-% Verdickungsmittel enthält, ausgewählt unter wasserdispergierbaren Polysacchariden, ausgewählt unter Senegal-Akaziengummi, Agar, Agarose, Biosaccharidgummi, Carrageen, Cellulose, Gellangummi, Guargummi, Hyaluronsäure, Pektin, Sklerotiumgummi und Xanthangummi; polymeren Verdickungsmitteln, ausgewählt unter Polyacrylaten, Polyacrylamiden, Polymethacrylat, Acrylat-Copolymer, Methacrylat-Copolymer, Acrylamid-Copolymer und Acryloyldimethyltaurat-Copolymer und Mischungen davon;
wobei die Zusammensetzung darin dispergierte Gasblasen aufweist;
wobei die Gasblasen 5 bis 35%, vorzugsweise 7 bis 30%, bevorzugter 10 bis 25% des Volumens der Zusammensetzung ausmachen;
wobei die Gasblasen aus Luft, Kohlendioxid, Stickstoff, Distickstoffmonoxid oder Mischungen davon bestehen; und
wobei die Zusammensetzung durch ein Verfahren erhalten wird, das die folgenden Schritte umfasst:
a. Herstellen einer Öl-in-Wasser-Emulsion durch Emulgieren der Ölphase in die wässrige Phase unter Bedingungen moderater Scherung bei Atmosphärendruck und einer Temperatur von 22°C bis 85°C; und
b. Einleiten des Gases in die Emulsion zur Belüftung der Zusammensetzung durch Aufschlag- oder Einspritztechniken bei 0,066 bis 2,10 atm (50 bis 1600 mmHg), vorzugsweise bei 0,079 bis 1,71 atm (60 bis 1300 mmHg), bevorzugter von 0,092 bis 1,315 atm (70 bis 1000 mmHg) und einer Temperatur von 22°C bis 85°C, in ein Rohr eines Durchmessers in dem Bereich von 1,27 bis 10,16 cm (0,5 bis 4 Zoll).

2. Kosmetische Zusammensetzung nach Anspruch 1, wobei das Polyol Glycerin, Butylenglykol, Sorbitol oder ein Gemisch davon umfasst und die kosmetische Zusammensetzung 0,1 bis 15%, vorzugsweise 1 bis 12% Polyol, bezogen auf das Gewicht der kosmetischen Zusammensetzung, umfasst.

3. Kosmetische Zusammensetzung nach einem der vorhergehenden Ansprüche, wobei die Stärke native Stärke, chemisch modifizierte Stärke oder eine Mischung davon ist.

4. Kosmetische Zusammensetzung nach einem der vorhergehenden Ansprüche, wobei die kosmetische Zusammensetzung 0,1 bis 3 Gew.-%, vorzugsweise 0,25 bis 2 Gew.-% Stärke in der kosmetischen Zusammensetzung umfasst.

5. Kosmetische Zusammensetzung nach einem der vorhergehenden Ansprüche, wobei die kosmetische Zusammensetzung 0,1 bis 10%, vorzugsweise 0,5 bis 8%, nichtionischen Emulgator, bezogen auf das Gewicht der kosmetischen Zusammensetzung, enthält und wobei der nichtionische Emulgator einen durchschnittlichen HLB-Wert von 7 bis 11 aufweist.

6. Kosmetische Zusammensetzung nach einem der vorhergehenden Ansprüche, wobei der nichtionische Emulgator Glycerylstearat, Glykolstearat und Stearamid AMP, PEG-100-Stearat, Cetylalkohol, Stearylalkohol oder Mischungen davon umfasst.

7. Kosmetische Zusammensetzung nach einem der vorhergehenden Ansprüche, wobei die wässrige Phase in einer Menge von 30 bis 80%, vorzugsweise von 35 bis 75%, bevorzugter von 40 bis 70%, bezogen auf das Gewicht der kosmetischen Zusammensetzung, vorliegt.

8. Kosmetische Zusammensetzung nach einem der vorhergehenden Ansprüche, wobei die Ölphase ein Öl, Butter, Wachs oder eine Mischung davon in einer Gesamtmenge in dem Bereich von 20 bis 50%, vorzugsweise von 25 bis 45%, bezogen auf das Gewicht der Zusammensetzung, umfasst.

9. Kosmetische Zusammensetzung nach einem der vorhergehenden Ansprüche, wobei die Ölphase Candelillawachs, Capryl/Caprinsäuretriglycerid, Carnaubawachs, Kakaobutter, Kokosnussöl, Jojobasamenöl, Sheabutter, Sonnenblumenöl oder Mischungen davon umfasst und mindestens 30%, vorzugsweise mindestens 40% der Ölphase bei Raumtemperatur fest sind.

## Revendications

1. Composition cosmétique sous la forme d'une émulsion huile dans l'eau aérée, la composition cosmétique comprenant :
a. un amidon ;
b. un polyol ;
c. un émulsifiant non-ionique ;
d. une phase aqueuse ; et
e. une phase huileuse,
dans laquelle le rapport en poids de la quantité totale d'émulsifiant à la phase huileuse de l'émulsion est de 1/6,5 à 1/3,75, de préférence de 1/6,25 à 1/3,8, mieux encore de 1/6 à 1/4 ;
dans laquelle la composition cosmétique a une masse volumique, quand elle est mesurée à 25 °C et sous la pression atmosphérique, située dans la plage allant de 0,5 à 1,3 g/cm³, de préférence de 0,6 à 1,1 g/cm³, de préférence de 0,7 à 1,0 g/cm³ ;
dans laquelle la composition cosmétique contient moins de 0,2 % en poids d'agent épaississant choisi parmi les polysaccharides dispersibles dans l'eau choisis parmi la gomme arabique, la gélose, l'agarose, une gomme de biosaccharide, la carraghénane, la cellulose, la gomme gellane, la gomme de guar, l'acide hyaluronique, la pectine, la gomme de sclérote, et la gomme xanthane ; les épaississants polymères choisis parmi les polyacrylates, les polyacrylamides, un polyméthacrylate, un copolymère d'acrylate, un copolymère de méthacrylate, un copolymère d'acrylamide, et un copolymère d'acryloyldiméthyltaurate ; et leurs mélanges ;
dans laquelle la composition a des bulles de gaz dispersées dans celle-ci ;
dans laquelle les bulles de gaz forment 5 à 35 %, de préférence 7 à 30 %, mieux encore 10 à 25 % du volume de la composition ;
dans laquelle les bulles de gaz sont des bulles d'air, de dioxyde de carbone, d'azote, d'oxyde nitreux, ou de mélanges de ceux-ci ;
et dans laquelle la composition est obtenue par un procédé comprenant les étapes de :
a. préparation d'une émulsion huile dans l'eau par émulsification de la phase huileuse dans la phase aqueuse dans des conditions de cisaillement modéré sous la pression atmosphérique et à une température de 22 °C à 85 °C ; et
b. introduction du gaz dans l'émulsion pour aérer la composition par des techniques de battage ou d'injection sous 0,066 à 2,10 atm (50 à 1 600 mm Hg), de préférence 0,079 à 1,71 atm (60 à 1 300 mm Hg), mieux encore 0,092 à 1,315 atm (70 à 1 000 mm Hg) et à une température de 22 °C à 85 °C, dans un tuyau ayant un diamètre situé dans la plage allant de 1,27 à 10,16 cm (0,5 à 4 pouces).

2. Composition cosmétique selon la revendication 1, dans laquelle le polyol comprend la glycérine, le butylèneglycol, le sorbitol, ou un mélange de ceux-ci, et dans laquelle la composition cosmétique comprend 0,1 à 15 %, de préférence 1 à 12 % de polyol en poids de la composition cosmétique.

3. Composition cosmétique selon l'une quelconque des revendications précédentes, dans laquelle l'amidon est un amidon natif, un amidon chimiquement modifié, ou un mélange de ceux-ci.

4. Composition cosmétique selon l'une quelconque des revendications précédentes, dans laquelle la composition cosmétique comprend 0,1 à 3 %, de préférence 0,25 à 2 % en poids d'amidon dans la composition cosmétique.

5. Composition cosmétique selon l'une quelconque des revendications précédentes, dans laquelle la composition cosmétique [comprend] 0,1 à 10 %, de préférence 0,5 à 8 % d'émulsifiant non-ionique en poids de la composition cosmétique, et dans laquelle l'émulsifiant non-ionique a un indice HLB moyen de 7 à 11.

6. Composition cosmétique selon l'une quelconque des revendications précédentes, dans laquelle l'émulsifiant non-ionique comprend le stéarate de glycéryle, le stéarate de glycol et le stéaramide AMP, le stéarate PEG-100, l'alcool cétylique, l'alcool stéarylique, ou des mélanges de ceux-ci.

7. Composition cosmétique selon l'une quelconque des revendications précédentes, dans laquelle la phase aqueuse est présente en une quantité comprise entre 30 et 80 %, de préférence entre 35 et 75 %, mieux encore entre 40 et 70 % en poids de la composition cosmétique.

8. Composition cosmétique selon l'une quelconque des revendications précédentes, dans laquelle la phase huileuse comprend une huile, un beurre, une cire, ou un mélange de ceux-ci en des quantités totales situées dans la plage allant de 20 à 50 %, de préférence de 25 à 45 % en poids de la composition.

9. Composition cosmétique selon l'une quelconque des revendications précédentes, dans laquelle la phase huileuse comprend la cire de candélilla, un triglycéride caprylique/caprique, la cire de carnauba, le beurre de cacao, l'huile de coco, l'huile de jojoba, le beurre de karité, l'huile de tournesol, ou des mélanges de ceux-ci, et dans laquelle au moins 30 %, de préférence au moins 40 % de la phase huileuse sont solides à la température ambiante.
